# EUROPÄISCHE PATENTSCHRIFT

(11) **EP 0 936 865 B1**
(45) Veröffentlichungstag und Bekanntmachung des Hinweises auf die Patenterteilung: **18.12.2002**
(21) Anmeldenummer: 97913170.3
(22) Anmeldetag: 22.10.1997
(51) Int. Cl.: A01N 37/46

(54) **STABILE WÄSSRIGE FORMULIERUNG VON 3-(N-BUTYLACETAMINO)-PROPIONSÄUREETHYLESTER**
STABLE AQUEOUS FORMULATION OF 3-(N-BUTYLACETAMINO)-PROPIONIC ACID ETHYL ESTER
FORMULATION AQUEUSE STABLE DE 3-(N-BUTYLACETAMINO)-ETHYLESTER D'ACIDE PROPIONIQUE

(30) Priorität: 02.11.1996 DE 19645250
(43) Veröffentlichungstag der Anmeldung: 25.08.1999
(73) Patentinhaber: MERCK PATENT GmbH, 64271 Darmstadt (DE)
(72) Erfinder: KURZ, Thekla, D-64285 Darmstadt (DE); HITZEL, Sabine, D-64409 Messel (DE); WILLE, Dorothee, D-64291 Darmstadt (DE); JALALIAN, Mohammad, D-64283 Darmstadt (DE)
(86) Internationale Anmeldenummer: EP9705826
(87) Internationale Veröffentlichungsnummer: WO98019537

(56) Entgegenhaltungen:
- EP-A- 0 717 982
- DE-A- 3 220 884
- DE-A- 3 220 885

## Beschreibung

Die vorliegende Erfindung betrifft eine stabile wäßrige Formulierung der Substanz 3-(N-Butylacetamino)-propionsäureethylester.

Die Substanz 3-(N-Butylacetamino)-propionsäureethylester hat bekanntermaßen eine insektenabwehrende Wirkung und wird auch als solches eingesetzt. Dieses Insekt- Repellent kann im Handel bezogen werden, z.B. von der Firma Merck KGaA, Darmstadt.

Insekten stellen für den Menschen in mancherlei Hinsicht eine Beeinträchtigung oder gar eine Bedrohung dar. Neben den durch einige Insektenarten angerichteten Schäden (z.B. die Vernichtung ganzer Ernten) werden sowohl der Mensch als auch viele Tiere von den verschiedensten Insekten auf die eine oder andere Weise belästigt, gestochen und anderweitig geplagt. Dabei kann es zu Infektionen und zur Übertragung von gefährlichen Krankheiten kommen.

Hauptwaffe im Kampf gegen die Insekten sind die Insektizide, deren Verwendung und Toxizitätsprofile aber nicht immer unproblematisch sind. Bei der Verhinderung der direkten Belästigung sind Maßnahmen wie der Einsatz von Insektenvernichtungsmitteln aber gar nicht unbedingt erforderlich.

Da heutzutage der Umweltschutz sehr im Vordergrund steht, werden bevorzugt vorbeugende Maßnahmen ergriffen.

Daher werden bei der Insektenbekämpfung nun eher Insektenabwehrmittel, sogenannte Repellentien, den Insektiziden vorgezogen, da diese lediglich darauf ausgerichtet sind, Insekten davon abzuhalten, sich auf einem Wirt niederzulassen.

Die Anforderungen an ein Insekt-Repellent sind hoch und vielfältig. Vor allem muß ein wirksamer Schutz der Haut vor Insekten gewährleistet sein. Die nachhaltige Abwehrwirkung über mehrere Stunden, auch unter klimatisch ungünstigen Bedingungen, ist wichtig. Das Repellent sollte eine möglichst breites Wirkungsspektrum besitzen.

Weitere Anforderungen sind eine maximale Haut- und Schleimhautverträglichkeit ohne toxische, allergisierende oder sensibilisierende Eigenschaften , wobei jedoch keine Hautpenetration erfolgen sollte.

Ferner sollte die Substanz eine hohe chemische Stabilität, d.h. keine Hydrolysierbarkeit, keine Photooxidierbarkeit, keine Oxidierbarkeit, hohe Thermostabilität und hohe Schweißfestigkeit aufweisen.

Weiterhin muß ein Insekt-Repellent mit den gängigen kosmetischen und pharmazeutischen Formulierungsgrundlagen gut verträglich und mischbar sein.

Das Insekt-Repellent 3-(N-Butytacetamino)propionsäureethylester erfüllt diese Vielzahl von Anforderungen.

Es ist jedoch festzustellen, daß diese Substanz in wäßrigen Formulierungen während der Lagerzeit abgebaut wird. Dieser Abbau ist unter dem Einfluß erhöhter Temperaturen beschleunigt. Schon eine Temperaturerhöhung um 10 °C kann eine Verdopplung der Abbaugeschwindigkeit bewirken.

Dies ist als Nachteil anzusehen, denn wie oft werden entsprechende Insektenabwehrmittel nach ihrer Herstellung und dem Verkauf über Jahre hinweg gelagert oder nur nach und nach über einen langen Zeitraum verbraucht. Außerdem treten häufig höhere Temperaturen (durchaus 50 °C und höher) bei der Lagerung von kosmetischen Formulierungen im Sommer auf, insbesondere in südlichen Ländern. Dabei kann es also vorkommen, daß das Insekt-Repellent schon so stark abgebaut wurde, daß die Wirkung erheblich vermindert.

Es erscheint somit wünschenswert, wäßrige oder wasserhaltige Formulierungen mit verbesserter Stabilität zur Verfügung zu stellen, welche über einen langen Zeitraum, auch bei erhöhten Temperaturen, einen unveränderten Gehalt an 3-(N-Butylacetamino)-propionsäureethylester aufweisen.

Überraschend wurde nun gefunden, daß die Stabilität wäßriger Formulierungen enthaltend 3-(N-Butylacetamino)propionsäureethylester erheblich erhöht werden kann, wenn Formulierungen mit höherem pH-Wert, bevorzugt gepufferte Lösungen, eingesetzt werden.

Basisformulierungen für kosmetische oder pharmazeutische Zubereitungen werden oft auf der Grundlage wäßrig-alkoholischer Lösungen hergestellt. Auch in Verbindung mit dem Insekt-Repellent 3-(N-Butylacetamino)-propionsäureethylester sind solche Formulierungen bekannt. Aus EP-A-0 717 982 ist eine Zusammensetzung bekannt, die 4-tButyl-4-methoxy-dibenzoylmethan und 3-(N-Butylacetamino)propionsäureethylester sowie 0,5 % Triethanolamin als Puffer enthält. Es finden sich jedoch in der Literatur keinerlei Hinweise, daß Formulierungen mit höherem pH-Wert, insbesondere gepufferte, zur Stabilitätsverbesserung eingesetzt werden.

Gegenstand der Erfindung ist eine stabile wäßrige Formulierung des Insekt-Repellents 3-(N-Butylacetamino)propionsäureethylester, die dadurch gekennzeichnet ist, daß gepufferte Lösungen mit einem pH-Wert größer 3.0, worin als Puffer vorzugsweise ein Puffersystem, ausgewählt aus einer Gruppe enthaltend die Puffersysteme Citronensäure/Na₂HPO₄, Tris(hydroxymethyl)amino-methan/HCl, Dinatriumcitrat/HCl, Kaliumhydrogenphthalat/NaOH, Dinatriumcitrat/NaOH, Kaliumdihydrogenphosphat/ Dinatriumhydrogenphosphat und Triethanolamin, Titriplex III/ HCl enthalten ist, zur Stabilisierung eingesetzt werden.

Der pH-Wert dieser Lösungen liegt vorzugsweise in einem Bereich von pH 3.5 bis 7.9.

Als Puffersysteme kommen alle in diesen pH-Wert-Bereich fallenden bekannten Pufferlösungen in Frage. Dem Fachmann sind solche Puffer allgemein bekannt und müssen hier nicht im Einzelnen näher beschrieben werden.

Erfindungsgemäß wird vorzugsweise ein Puffer, ausgewählt aus der folgenden Gruppe von Puffersystemen, eingesetzt. Dabei soll diese Gruppe von Puffersystemen hier nur beispielhaft aufgezählt werden und keinerlei limitierenden Charakter auf die erfindungsgemäßen Formulierungen haben:

Citronensäure/Dinatriumhydrogenphosphat, wirksam in einem pH-Bereich 2.2 bis 7.8 (vorzugsweise 0.1 M Citronensäure/0.2 M Na₂HPO₄ ), Tris(-hydroxymethyl)-aminomethan/HCl, wirksam in dem pH-Bereich um 7.2 bis 9.0 (vorzugsweise 0.2 M/0.1 M), Dinatriumcitrat/HCl, wirksam im Bereich pH 1.2 bis 5.0 (vorzugsweise 0.1 M/0.1 M), Kaliumhydrogenphthalat/NaOH, wirksam im Bereich pH 4.2 bis 6.2 (vorzugsweise 0.1 M/ 0.1 M), Dinatriumcitrat/NaOH, wirksam im Bereich pH 5.2 bis 6.6 (vorzugsweise 0.1 M/0.1M), Kaliumdihydrogenphosphat/Dinatriumhydrogenphosphat, wirksam im Bereich pH 5.0 bis 8.0 (vorzugsweise beide 1/15 mol/L), oder auch Triethanolamin + Titriplex III/HCl, wirksam im Bereich pH 7.0 bis 8.8 (vorzugsweise 0.5 mol/L / 0.05 mol/L).

Gegenstand der Erfindung ist auch die Verwendung von Lösungen mit einem pH-Wertes größer pH 3.0, vorzugsweise gepufferte Lösungen, worin als Puffer vorzugsweise ein Puffersystem, ausgewählt aus einer Gruppe enthaltend die Puffersysteme Citronensäure/Na₂HPO₄, Tris(hydroxymethyl)amino-methan/HCI, Dinatriumcitrat/HCl, Kaliumhydrogenphthalat/NaOH, Dinatriumcitrat/NaOH, Kaliumdihydrogenphosphat/ Dinatriumhydrogenphosphat und Triethanolamin, Titriplex III/ HCl enthalten ist, zur Stabilisierung wasserhaltiger Formulierungen des Insekt-Repellents 3-(N-Butylacetamino)-propionsäureethylester. Der pH-Wert dieser Lösungen liegt in einem Bereich von pH größer 3.0. Vorzugsweise liegt der pH-Wert im Bereich von pH 3.5 bis 7.9.

Als wasserhaltige Formulierungen kommen dabei u.a. beispielsweise Lotionen, Emulsionen, wie als Creme oder als Milch (W/O oder O/W), Gele oder auch feste Stifte in Frage.

In diese wasserhaltigen Formulierungen können neben anderen Zusätzen auch UV-Filter eingearbeitet werden, wodurch Sonnenschutzmittel oder gegen UV-Stahlung schützende Formulierungen erhalten werden, die auf die Haut aber gegebenenfalls auch auf die Haare aufgetragen werden können. Beispiele geeigneter und im Handel erhältlicher UV-Filter sind u. a. Eusolex 6300®, Eusolex 4300®, Eusolex 9020®, Eusolex 232®, Eusolex 2292®, Eusolex OCR®, Eusolex HMS®, Eusolex 6007®, PABA oder Uvinul T 150®. In den erfindungsgemäßen Formulierungen können geeignete UV-Filter einzeln oder in Kombination mit anderen in Konzentrationen von 0,5 bis 10 Gew.-% eingearbeitet sein.

Eine erfindungsgemäße Emulsion, welche als Schutzcreme oder - milch gegen Insekten vorliegt, kann außer der insektenabwehrenden Substanz 3-(N-Butylacetamino)-propionsäureethylester in Lösung bzw. in Lösung mit einem geeigneten Puffer auch beispielsweise folgende Substanzen enthalten:

Fettalkohole, Fettsäureester, Fettsäuren, Lanolin, natürliche oder synthetische Öle oder Wachse und Emulgatoren in Anwesenheit von Wasser.

Erfindungsgemäße Lotionen enthalten u.a. natürliche oder synthetische Öle und Wachse, Lanolin, Fettsäureester, Niedrigalkohole oder Polyole.

Bei Gelen sind meist noch zusätzlich Verdickungsmittel, wie z.B. Kieselerde, enthalten.

Die festen Stifte bestehen aus natürlichen oder synthetischen Wachsen und Ölen, Fettalkoholen, Fettsäuren, Fettsäureester, Lanolin, neben den erfindungsgemäßen Zutaten.

Auch erfindungsgemäße Formulierungen als Aerosole sind möglich, dabei verwendet man in der Regel die üblichen Treibmittel, wie Alkane, Fluoralkane und Chlorfluoralkane.

Die Zusammensetzung und Herstellung solcher wasserhaltiger Formulierungen sind dem Fachmann allgemein bekannt und müssen hier nicht näher erläutert werden.

In einer weiteren bevorzugten Ausführungsform liegt der pH-Wert in den erfindungsgemäßen Formulierungen im Bereich von 4.5 bis 8.0 und einen ganz besonders starken Stabilitätseffekt weisen die Formulierungen mit einem pH-Wert zwischen 6.5 und 7.9 auf.

Der Gehalt an 3-(N-Butylacetamino)propionsäureethylester in den erfindungsgemäßen Formulierungen beträgt 1 bis 50 Gew. %, vorzugsweise 10 bis 30 Gew. %.

Die erfindungsgemäßen Formulierungen werden einfach durch Mischen der einzelnen Komponenten unter Rühren hergestellt.

Mit der Bereitstellung der erfindungsgemäßen Formulierungen stehen nun wäßrige bzw. wasserhaltige Formulierungen mit 3-(N-Butylacetamino)-propionsäureethylester zur Verfügung, die über einen langen Zeitraum einen unveränderten Gehalt an dieser Substanz aufweisen. Selbst bei Lagertemperaturen von 30° bis über 40° C findet bei den bevorzugten höheren pH-Werten praktisch keine Zersetzung statt.

Auch ohne weitere Ausführungen wird davon ausgegangen, daß ein Fachmann die obige Beschreibung in weitestem Umfang nutzen kann. Die bevorzugten Ausführungsformen sind deswegen lediglich als beschreibende, keineswegs als in irgendeine Weise limitierte Offenbarung aufzufassen.

Die vollständige Offenbarung aller vor- und nachstehend aufgeführten Anmeldungen, Patente und Veröffentlichungen sind durch Bezugnahme in diese Anmeldung eingeführt.

Zum besseren Verständnis und zur Verdeutlichung werden im folgenden Beispiele gegeben, die im Rahmen des Schutzbereichs der vorliegenden Erfindung liegen, nicht jedoch geeignet sind, die Erfindung auf diese Beispiele zu beschränken.

### Beispiel 1

Mit dem Puffersystem 0.1 M Citronensäure / 0.2 M Natriumsulfat werden durch einfaches Mischen der Komponenten unter Rühren Lösungen von pH 3.5, 4.4 und 6.5 hergestellt. Dieses Puffersystem kann also über einen breiten pH-Bereich wirksam sein.

| a) Man stellt eine Lösung von pH 3.5 her, mit folgender Zusammensetzung (in Gew. %): | | |
|---|---|---|
| | | |
| 3-(N-Butylacetamino)propionsäureethylester (Art.-Nr. 111887) | (1) | 10.0% |
| Ethanol 96 % (Art.-Nr. 100971) | (1) | 15.0 % |
| Puffer pH 3.0 | | ad 100.0 % |

| b) Man stellt eine Lösung von pH 4.4 her, mit folgender Zusammensetzung (in Gew. %): | | |
|---|---|---|
| | | |
| 3-(N-Butylacetamino)propionsäureethylester (Art.-Nr. 111887) | (1) | 10.0 % |
| Ethanol 96 % (Art.-Nr. 100971) | (1) | 15.0 % |
| Puffer pH 4.0 | | ad 100.0 % |

| c) Man stellt eine Lösung von pH 6.5 her, mit folgender Zusammensetzung (in Gew. %): | | |
|---|---|---|
| | | |
| 3-(N-Butylacetamino)propionsäureethylester (Art.-Nr. 111887) | (1) | 10.0 % |
| Ethanol 96 % (Art.-Nr. 100971) | (1) | 15.0% |
| Puffer pH 6.0 | | ad 100.0% |

| | | |
|---|---|---|
| Bezugsquelle: (1) Merck KGaA, Darmstadt | | |

### Beispiel 2

Mit dem Puffersystem 0.2 M Tris(hydroxymethyl)aminomethan / 0.1 M HCl wird eine Lösung mit pH 7.9 durch Zusammengeben folgender Komponenten unter Rühren hergestellt (Angaben in Gew. %):

| | | |
|---|---|---|
| 3-(N-Butylacetamino)propionsäureethylester (Art.-Nr. 111887) | (1) | 10.0 % |
| Ethanol 96 % (Art.-Nr. 100971) | (1) | 15.0 % |
| Puffer pH 8,0 | | ad 100.0 % |

| | | |
|---|---|---|
| Bezugsquelle: (1) Merck KGaA. Darmstadt | | |

### Beispiel A

Die Stabilität von 3-(N-Butylacetamino)propionsäureethylester wurde in Abhängigkeit des pH-Wertes in gepufferten Lösungen überprüft. Die erfindungsgemäßen wäßrigen Formulierungen, hergestellt nach den Beispielen 1 und 2, wurden einem Lagerungstest über 12 Monate sowohl bei Raumtemperatur als auch bei 40 °C unterzogen.

Die Ergebnisse sind in Tabellen 1 und 2 zu lesen.

**Tabelle 1**

| pH-Wert der gepufferten Lösung | Lagerung bei Raumtemperatur [Monate] | | | | |
|---|---|---|---|---|---|
| | 0 | 3 | 6 | 9 | 12 |
| | Gehalt Repellent in % | | | | |
| 3.5 | 10.1 | 8.8 | 8.0 | 7.2 | 6.4 |
| 4.5 | 10.2 | 9.8 | 9.6 | 9.4 | 9.0 |
| 6.5 | 10.2 | 9.7 | 9.6 | 9.7 | 9.5 |
| 7.9 | 10.2 | 9.7 | 9.6 | 9.7 | 9.5 |

**Tabelle 2**

| pH-Wert der gepufferten Lösung | Lagerung bei 40 °C Raumtemperatur [Monate] | | | | |
|---|---|---|---|---|---|
| | 0 | 3 | 6 | 9 | 12 |
| | Gehalt Repellent in % | | | | |
| 3.5 | 10.1 | 5.8 | 3,5 | 2.8 | 2.7 |
| 4.5 | 10.2 | 8.2 | 7.0 | 5.3 | 4.0 |
| 6.5 | 10.2 | 9.3 | 8.5 | 7.8 | 6.8 |
| 7.9 | 10.2 | 9.6 | 9.3 | 8.9 | 7.9 |

### Beispiel B

In diesem Beispiel wurde die wäßrige Lösungen von 3-(N-Butylacetamino)-propionsäureethylester einem Lagerungstest bei 40 °C in Abhängigkeit vom pH-Wert unterzogen. Die entsprechenden wäßrigen Lösungen setzen sich folgendermaßen zusammen:

### Lösung 1 (pH 4,5):

| | | in Gew.-% |
|---|---|---|
| 3-(N-Butylacetamino)propionsäureethylester (Art.-Nr. 111887) | (1) | 10.0 |
| Ethanol 96 % (Art.-Nr. 100971) | (1) | 15.0 |
| NaCI (Art.-Nr.6400) | (1) | 0.1 |
| Wasser, demin. pH-Einstellung mit Salzsäure | | ad 100.0 |

### Lösung 2 (pH 6):

Zusammensetzung wie Lösung 1, pH-Wert-Einstellung auf pH 6 mit Natronlauge.

### Lösung 3 (pH 8):

Zusammensetzung analog Lösung 1; pH-Einstellung auf pH 8 mit Natronlauge.
Bezugsquelle:
Merck KGaA, Darmstadt

In Tabelle 3 sind nun die Ergebnisse des Lagerungstestes der verschiedenen Lösungen zusammengefaßt.

**Tabelle 3**

| pH-Wert der gepufferten Lösung | Lagerung bei Raumtemperatur [Monate] | | | | |
|---|---|---|---|---|---|
| | 0 | 3 | 6 | 9 | 12 |
| | Gehalt Repellent in % | | | | |
| (1) 4.5 | 9.9 | 4.4 | 2.7 | 2.2 | 2.5 |
| (2) 6.0 | 10.1 | 6.0 | 2.9 | 2.5 | 2.6 |
| (3) 8.0 | 10.0 | 8.6 | 3.0 | 2.5 | 2.6 |

Vergleicht man nun die Tabellen 2 und 3 so ist klar ersichtlich, daß das Insekt-Repellent in den erfindungsgemäßen gepufferten Formulierungen erheblich stabiler ist, der Abbau deutlich verlangsamt ist, insbesondere bei höheren pH-Werten.

## Patentansprüche

1. Stabile wäßrige Formulierung des Insekt-Repellents 3-(N-Butylacetamino)-propionsäureethylester,
**dadurch gekennzeichnet, daß** gepufferte Lösungen mit einem pH-Wert größer 3.0,
worin als Puffer vorzugsweise ein Puffersystem, ausgewählt aus einer Gruppe enthaltend die Puffersysteme Citronensäure/Na₂HPO₄, Tris(hydroxymethyl)amino-methan/HCl, Dinatriumcitrat/HCl, Kaliumhydrogenphthalat/NaOH, Dinatriumcitrat/NaOH, Kaliumdihydrogenphosphat/ Dinatriumhydrogenphosphat und Triethanolamin, Titriplex III/ HCl enthalten ist,
zur Stabilisierung eingesetzt werden.

2. Formulierung nach Anspruch 1, **dadurch gekennzeichnet, daß** der pH-Wert in einem Bereich von pH 3.5 bis 7.9 liegt.

3. Formulierung nach einem der Ansprüche 1 bis 2, **dadurch gekennzeichnet, daß** das Insekt-Repellent in einer Konzentration von 1 Gew.-% bis 50 Gew.-%, vorzugsweise von 10 bis 30 Gew.-%, vorliegt.

4. Wasserhaltige Formulierung gemäß einem oder mehreren der Ansprüche 1 bis 3, enthaltend einen oder mehrere UV-Filter in Kombination in Konzentrationen von 0,5 bis 10 Gew.-%.

5. Verwendung von Lösungen mit einem pH-Wertes größer pH 3.0, vorzugsweise gepufferte Lösungen, worin als Puffer vorzugsweise ein Puffersystem, ausgewählt aus einer Gruppe enthaltend die Puffersysteme Citronensäure/Na₂HPO₄, Tris(hydroxymethyl)aminomethan/HCl, Dinatriumcitrat/HCl, Kaliumhydrogenphthalat/NaOH, Dinatriumcitrat/NaOH, Kaliumdihydrogenphosphat/ Dinatriumhydrogenphosphat und Triethanolamin, Titriplex III/ HCI enthalten ist, zur Stabilisierung wasserhaltiger Formulierungen des Insekt-Repellents 3-(N-Butylacetamino)-propionsäureethylester.

6. Verwendung nach Anspruch 5, **dadurch gekennzeichnet, daß** der pH-Wert dieser Lösungen in einem Bereich von pH 3.5 bis 7.9 liegt.

7. Verwendung nach einem der Ansprüche 5 bis 6, **dadurch gekennzeichnet, daß** das Insekt-Repellent in einer Konzentration von 1 Gew.-% bis 50 Gew.-%, vorzugsweise von 10 bis 30 Gew. %, enthalten ist.

8. Verwendung von wasserhaltigen Formulierungen gemäß einem oder mehreren der Ansprüche 1 bis 4 zur Herstellung von Formulierungen in Form von Lotionen, Emulsionen, Cremes oder Milch, Gelen oder festen Stiften.

## Claims

1. Stable aqueous formulation of the insect repellent ethyl 3-(N-butylacetamino)propionate, **characterized in that** buffered solutions having a pH greater than 3.0, in which the buffer present is preferably a buffer system, selected from a group consisting of the buffer systems citric acid/Na₂HPO₄, tris (hydroxymethyl) aminomethane/HCl, disodium citrate/HCl, potassium hydrogen-phthalate/NaOH, disodium citrate/NaOH, potassium dihydrogenphosphate/disodium hydrogenphosphate and triethanolamine and Titriplex III/HCl, are used for stabilization.

2. Formulation according to Claim 1, **characterized in that** the pH is in a range from pH 3.5 to 7.9.

3. Formulation according to one of Claims 1 to 2, **characterized in that** the insect repellent is present in a concentration of from 1% by weight to 50% by weight, preferably from 10 to 30% by weight.

4. Hydrous formulation according to one or more of Claims 1 to 3, comprising one or more UV filters in combination in concentrations of from 0.5 to 10% by weight.

5. Use of solutions having a pH greater than pH 3.0, preferably buffered solutions, in which the buffer present is preferably a buffer system, selected from a group consisting of the buffer systems citric acid/Na₂HPO₄, tris (hydroxymethyl) aminomethane/ HCl, disodium citrate/HCl, potassium hydrogenphthalate/NaOH, disodium citrate/NaOH, potassium dihydrogenphosphate/disodium hydrogenphosphate and triethanolamine and Titriplex III/HCl, for stabilizing hydrous formulations of the insect repellent ethyl 3-(N-butylacetamino)propionate.

6. Use according to Claim 5, **characterized in that** the pH of these solutions is in a range from pH 3.5 to 7.9.

7. Use according to one of Claims 5 to 6, **characterized in that** the insect repellent is present in a concentration of from 1% by weight to 50% by weight, preferably from 10 to 30% by weight.

8. Use of hydrous formulations according to one or more of Claims 1 to 4 for preparing formulations in the form of lotions, emulsions, creams or milk, gels or solid sticks.

## Revendications

1. Formulation aqueuse stable du répulsif pour insectes l'ester éthylique de l'acide 3-(N-butylacétamido)propionique, **caractérisée en ce que** l'on utilise pour la stabilisation des solutions tamponnées ayant un pH supérieur à 3,0, contenant, comme tampons, de préférence un système de tampons choisis dans le groupe constitué par les systèmes de tampons acide citrique/Na₂HPO₄, Tris(hydroxyméthyl)aminométhane/HCl, citrate disodique/HCl, hydrogénophtalate de potassium/NaOH, citrate disodique/NaOH, dihydrogénophosphate de potassium/hydrogénophosphate disodique et triéthanolamine, tritriplex III/HCl.

2. Formulation selon la revendication 1, **caractérisée en ce que** le pH est situé dans un intervalle compris entre 3,5 et 7,9.

3. Formulation selon l'une des revendications 1 et 2, **caractérisée en ce que** le répulsif pour insectes est présent à une concentration comprise entre 1 % en poids et 50 % en poids, de préférence entre 10 et 30 % en poids.

4. Formulation aqueuse selon l'une ou plusieurs des revendications 1 à 3, contenant un ou plusieurs filtres UV en combinaison à des concentrations comprises entre 0,5 et 10 % en poids.

5. Utilisation des solutions ayant un pH supérieur à 3,0, de préférence des solutions tamponnées contenant, comme tampons, de préférence un système de tampons choisi dans le groupe constitué par les systèmes de tampons acide citrique/Na₂HPO₄, Tris(hydroxyméthyl)amino-méthane/HCl, citrate disodique/HCl, hydrogénophtalate de potassium/NaOH, citrate disodique/NaOH, dihydrogénophosphate de potassium/hydrogéno-phosphate disodique et triéthanolamine, tritriplex III/HCl, pour la stabilisation de formulations aqueuses du répulsif pour insectes l'ester éthylique de l'acide 3-(N-butylacétamido)propionique.

6. Utilisation selon la revendication 5, **caractérisée en ce que** le pH de ces solutions est situé dans un intervalle compris entre 3,5 et 7,9.

7. Utilisation selon l'une des revendications 5 à 6, **caractérisée en ce que** le répulsif pour insectes est présent à une concentration comprise entre 1 % en poids et 50 % en poids, de préférence entre 10 % et 30 % en poids.

8. Utilisation des formulations aqueuses selon l'une des revendications 1 à 4 pour la fabrication de formulations sous forme de lotions, d'émulsions, de crèmes ou de lait, de gels ou de crayons solides.
